(19) Europäisches Patentamt
European Patent Office
Office européen des brevets

(11) Publication number: **0 111 151 B1**

# (12) EUROPEAN PATENT SPECIFICATION

(45) Date of publication of patent specification: **09.12.87**

(51) Int. Cl.[4]: **C 07 D 241/16,** C 07 D 241/20, C 07 D 241/26

(21) Application number: **83110974.9**

(22) Date of filing: **03.11.83**

## (54) Substituted nitropyrazine compounds, process for their preparation and pharmaceutical compositions containing them.

(30) Priority: **12.11.82 US 441203**

(43) Date of publication of application: **20.06.84 Bulletin 84/25**

(45) Publication of the grant of the patent: **09.12.87 Bulletin 87/50**

(84) Designated Contracting States: **CH DE FR GB IT LI NL**

(56) References cited: **US-A-3 660 397**

(73) Proprietor: **MERCK & CO. INC.**
**126, East Lincoln Avenue P.O. Box 2000**
**Rahway New Jersey 07065-0900 (US)**

(72) Inventor: **Hartmann, George D.**
**1529 Tennis Circle**
**Lansdale, PA 19446 (US)**

(74) Representative: **Blum, Rudolf Emil Ernst et al**
**c/o E. Blum & Co Patentanwälte Vorderberg 11**
**CH-8044 Zürich (CH)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 111 151 B1

**Description**

Title of the Invention

Substituted nitropyrazine compounds, process for their preparation and pharmaceutical compositions containing them.

Background of the Invention

This invention relates to 2 and/or 6 substituted 3-nitropyrazine compounds used as sensitizers of tumor cells to therapeutic radiation. It also relates to the process of preparing such compounds starting with chloroaminopyrazines, converting said chloroaminopyrazines to the corresponding chloronitropyrazines and aminating said chloronitropyrazines to produce the substituted nitropyrazines useful in the sensitization of tumor cells to the therapeutic effect of radiation.

At the present time, certain other unrelated compounds are in experimental clinical use as radiation sensitizers. However, these compounds — for example, metronidazole and misonidazole — suffer from the drawback that they also cause neurotoxicity which limits their usefulness. The compounds of the present invention are effective radiation sensitizers, but are believed to have a more favorable therapeutic ratio.

Detailed Description of the Invention

One object of the invention are nitropyrazine compounds of the formula I

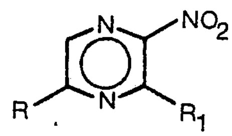

I

wherein R and $R_1$ are each selected from

a) hydrogen;

b) a carboalkoxy group in which the alkoxy group contains from 1—3 carbon atoms;

c) a group having the formula

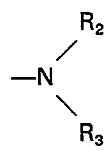

wherein

$R_2$ and $R_3$ are each selected from hydrogen and lower alkyl substituents of from 1—6 carbon atoms, said alkyl substituents having from 1—4 of the alkyl carbons substituted with a hydroxy or methoxy substituent and

d) halogen provided that at least one of the radicals R and $R_1$ is halogen or one of the specified amino groups.

Preferred new nitropyrazine compounds of formula Ia are compounds having the formula Ia

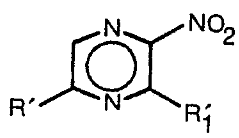

Ia

wherein R′ and $R'_1$ are selected from

a) hydrogen;

b) carboalkoxy in which the alkoxy group contains from 1—3 carbon atoms;

c) a group of formula

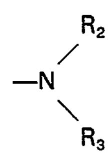

wherein

$R_2$ and $R_3$ are each selected from hydrogen and lower alkyl substituents of from 1 to 6 carbon atoms, said alkyl substituent having from 1 to 4 of the alkyl carbons substituted with a hydroxy or methoxy

substituent wherein a hydroxy substituent is preferred, provided that at least one of the radicals R′ and $R'_1$ is one of the specified amino groups.

The compounds of formula Ia are useful as radiation sensitizers.

A further object of the present invention accordingly is a pharmaceutical composition for enhancing the therapeutic effect of radiation which is characterized in that it contains as pharmaceutically active ingredient a compound of the above stated formula Ia.

Examples of nitropyrazine compounds of the above stated formula Ia are the following compounds:

2-(2,3-dihydroxypropyl)amino-3-nitropyrazine,

2-(2,3-dihdyroxypropyl)amino-5-nitropyrazine,

3-(5-nitropyrazin-2-yl)amino-1,2,4-butanetriol, and methyl 6-(2,3-dihydroxypropyl)amino-3-nitro-pyrazinoate.

A further group of compounds of formula I are those having the following formula Ib

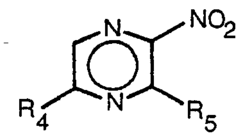

Ib

wherein

$R_4$ and $R_5$ are each selected from hdyrogen, halogen and carboalkoxy in which the alkoxy group contains from 1—3 carbon atoms, provided that at least one of the radicals $R_4$ and $R_5$ is halogen.

In preferred compounds of formula Ib the halogen is chloro. Preferred examples of compounds of formula Ib are the following:

3-chloro-2-nitropyrazine,

2-chloro-5-nitropyrazine and

methyl 6-chloro-3-nitropyrazinoate.

A further object of the present invention if a process for the preparation of the compounds of formula I, wherein R and $R_1$ are as defined before, which process is characterized in that a substituted aminopyrazine of the formula III

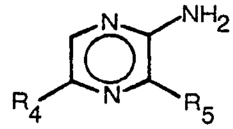

III

wherein

$R_4$ and $R_5$ are each selected from hydrogen, halogen and carboalkoxy groups, in which the alkoxy group contains from 1—3 carbon atoms provided that at least one of the radicals $R_4$ and $R_5$ is halogen, is treated with dimethyl sulfoxide and trifluoromethane sulfonic anhydride to produce a sulfinile compound of the formula IV

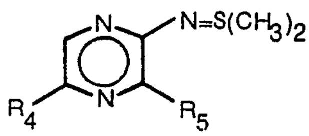

IV

which is oxidized to produce a nitropyrazine compound of formula Ib

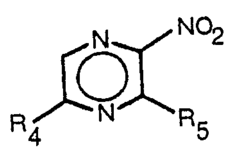

Ib

wherein

$R_4$ and $R_5$ have the same meaning as in formula III and said nitropyrazine compound of formula Ib is isolated or further reacted with ammonia or a substituted amine of formula II

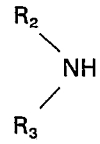

wherein

$R_2$ and $R_3$ are each selected from hydrogen and lower alkyl substituents of from 1—6 carbon atoms, said alkyl substituent having from 1—4 of the alkyl carbons substituted with a hydroxy or methoxy substituent, to produce a compound of formula I in which at least one of the substituents R and $R_1$ is a group of formula

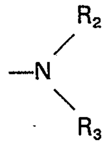

wherein

$R_2$ and $R_3$ have the same meaning as in formula II.

In said process in the first reaction step the aminopyrazine of formula III is preferably reacted with a corresponding reagent which is prepared by mixing dimethyl-sulfoxide in methylene chloride and cooled to −78°C. To the cooled solution is added trifluoromethane sulfonic anhdyride in a ratio of approximately 3 moles dimethylsulfoxide to 2 moles of anhydride to produce dimethyl sulfide ditriflate of the formula VI

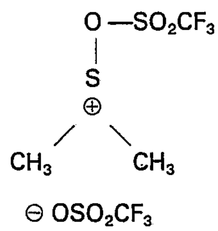

VI

Then, there is added to the cooled solution of VI the selected amino pyrazine of formula III while maintaining the temperature of the reaction mixture at −78°C to produce the sulfilimine of formula IV. The resulting suspension is made alkaline with aqueous sodium bicarbonate or sodium hydroxide. Additional methylene chloride is added and the product extracted into the methylene chloride phase which is separated and evaporated to remove the volatile organic solvent leaving the sulfilimine as a residue.

The product is then oxidized while maintaining the temperature at or below 0°C preferably using an organic peroxy carboxylic acid e.g., m-chloroperbenzoic acid as the oxidixing agent to produce the desired nitropyrazine of formula V.

Preferably, compounds of formula V wherein $R_4$ or $R_5$ is halogen are then treated with ammonia or an hydroxyalkylamine or an alkoxyalkylamine of the formula

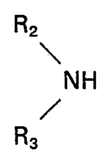

wherein

$R_2$ and $R_3$ are as defined hereinabove to produce a nitropyrazine of the formula I

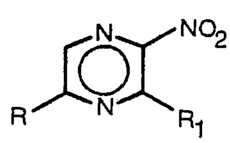

wherein at least one of R and $R_1$ is one of the specified amino groups.

The method of treatment of human patients or domestic animals undergoing radiation treatment of malignant disease processes employs the compounds of the present invention in pharmaceutical compositions that are administered orally or parenterally, or intraveneously. The dose employed depends

on the radiation protocol for each individual patient. In protocols where the radiation dose is divided into a large number of fractions, the drug can be administered at intervals in the schedule and not necessarily with each radiation treatment. It should be noted that the compounds of the present invention are not intended for chronic administration. In general, the drug is administered from 10 minutes to 5 hours prior to the radiation treatment in a dosage amount of between 0.25 to about 4.0 grams per square meter of body surface approximately equivalent to a dosage of 6 to 100 mg/kg of patient body weight as set forth in the "Nelson Textbook of Pediatrics" Eleventh Edition (1979) p. 31, edited by Vaughan, McKay, Behrman, and Nelson.

The dosage range given is the effective dosage range and the decision as to the exact dosage used must be made by the administering physician based on his judgement of the patient's general physical condition. In determining the dose for the individual patient, the physician may begin with an initial dose of 0.25 g/square meter of body surface to determine how well the drug is tolerated and increase the dosage with each succeeding radiation treatment, observing the patient carefully for any drug side effect. The composition to be administered is an effective amount of the active compound and a pharmaceutical carrier for said active compound.

The dosage form for intravenous administration is a sterile isotonic solution of the drug. Oral dosage forms such as tablets, capsules, or elixirs are preferred.

Capsules or tablets containing 25, 50, 100 or 500 mg of drug/capsule or tablets are satisfactory for use in the method of treatment of our invention.

The following examples are intended to illustrate the process of preparation, product and compositions. Temperature are in degrees celsius unless otherwise indicated throughout the application.

Example 1

3-Chloro-2-nitropyrazine

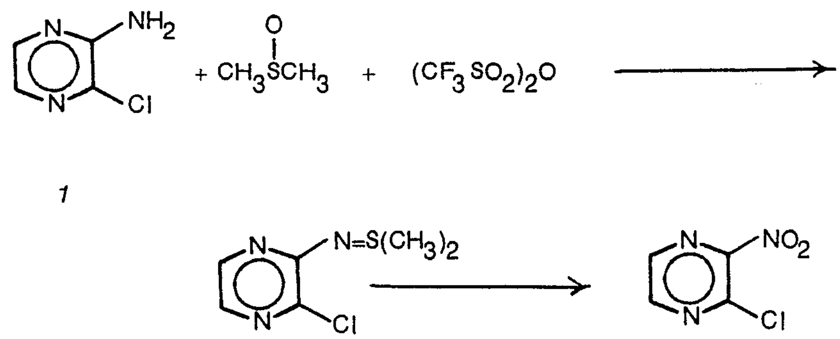

1

2

3

*Step A:* S,S-Dimethyl-N-(3-chloropyrazin-2-yl)sulfilimine (2)

To a mechanically stirred solution of 3.9 g (0.05 mol) dimethyl sufloxide in 30 ml dry methylene chloride at −78° under nitrogen is added 13.1 g (0.046 mol) trifluoromethanesulfonic anhydride dropwise to afford a white precipitate. To this is added a solution of 5.0 g (0.039 mol) 2-amino-3-chloropyrazine (*1*) in 30 ml methylene chloride/15 ml dimethyl sulfoxide and the resulting solution is stirred at −78° for 2 hours and at −55° for 1 hour. The reaction mixture is then quenched with 50 ml of 5% aqueous sodium bicarbonate solution and stirred at −5° for 5 minutes. The reaction mixture is diluted with 200 ml methylene chloride and the phases are separated. The aqueous phase is extracted with 250 ml methylene chloride and the combined organic phases are washed with 3 × 75 ml portions of water and dried over anhydrous sodium sulfate. The solvent is removed from the rotary evaporator to give 5.8 g (79%) of *2* as yellow crystals, m.p. 106—108°.

*Step B*: 3-Chloro-2-nitropyrazine (3)

To 7.9 g (0.46 mol, 80—90%) *m*-chloroperbenzoic acid in 70 ml methylene chloride cooled to −5° and stirred mechanically is added a solution of 5.36 g (0.028 mol) *2* in 30 ml methylene chloride dropwise at such a rate that the temperature does not exceed 0°. The reaction mixture is stirred at 0° for 40 minutes and then 3 ml of dimethyl sulfide is added with stirring for another 10 minutes at 0°. The cold reaction mixture is filtered quickly to afford a clear, green solution of the nitroso derivative. This solution is cooled to 0° and ozone is bubbled through until the solution is nearly colorless. The resulting suspension is extracted with 2 × 50 ml saturated sodium bicarbonate and the organic phase is dried over anhydrous sodium sulfate. The solvent is removed from the rotary evaporator to give a yellow oil which is purified by flash chromatography on silica gel with chloroform elution. Pure *3* is a pungent, yellow oil.

Example 2
2-Chloro-5-nitropyrazine

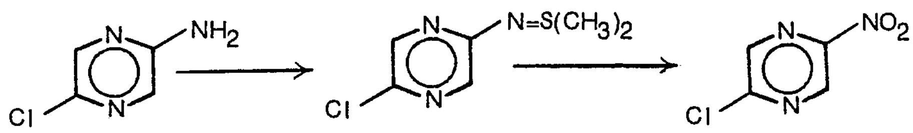

4 5 6

*Step A:* S,S-Dimethyl-N-(5-chloropyrazin-2-yl)sulfilimine (5)

Compound *5* is prepared from 2-amino-5-chloropyrazine as described above for *2* in Example 1A to afford pure *5* as a smelly, tan solid, m.p. 119—120°.

*Step B:* 2-Chloro-5-nitropyrazine (6)

Compound *6* is prepared as described in Example 1B for *3*, to afford pure *6* as yellow crystals, m.p. 90—92.

Example 3
Methyl-6-chloro-3-nitropyrazinoate 9

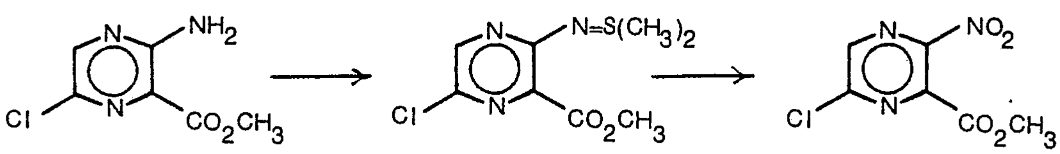

7 8 9

*Step A*: S,S-Dimethyl-N-(5-chloro-3-methoxycarbonylpyrazin-2-yl)sulfilimine (8)

Compound *8* is prepared from methyl 6-chloro-3-aminopyrazinoate (*7*) as described in Example 1A for *2*, to afford *8* which is recrystallized from methylene chloride/hexane. Pure *8* is obtained as a yellow solid, m.p. 167—9°.

*Step B*: Methyl 6-chloro-3-nitropyrazinoate (9)

Compound *9* is prepared from *8* as described in Example 1B for *3*, to give crude *9* which is purified by flash chromatography on silica gel eluting with 2% methanol/chloroform. Pure *9* is a pungent yellow oil.

Example 4
2-(2,3-Dihydroxypropyl)amino-3-nitropyrazine

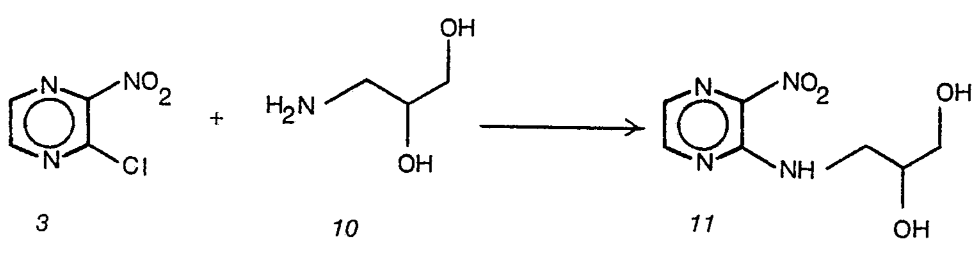

3 10 11

2-(2,3-Dihdyroxypropyl)amino-3-nitropyrazine (11)

To a solution of 1.5 g (0.0094 mol) *3* and 1.01 g (0.01 mol) triethylamine in 25 ml isopropanol is added 0.91 g (0.01 mol) 3-amino-1,2-propanediol (*10*). The reaction mixture becomes reddish and is stirred at room temperature for 16 hours. The solvent is then removed on the rotary evaporator and the residue is purified by flash chromatography on silica gel to afford pure *11* as yellow solid, m.p. 100—102°.

Example 5

2-(2,3-Dihydroxypropyl)amino-5-nitropyrazine

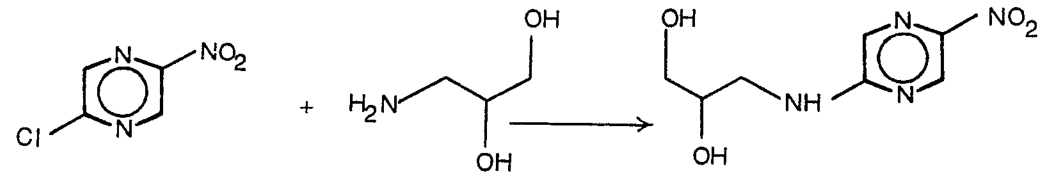

6 10 12

2-(2,3-Dihdyroxypropyl)amino-5-nitropyrazine (12)

To a solution of 0.5 g (0.003 mol) *6* and 0.3 g (0.003 mol) triethylamine in 10 ml acetonitrile is added 0.32 g (0.0035 mol) 3-amino-1,2-propanediol (*10*) in 10 ml iso-propanol. After stirring for 18 hours at room temperature the solvent is removed on the rotary evaporator and the residue is purified by flash chromatography on silica gel eluting with 10% methanol/chloroform. Pure *12* as Rf 0.4 and is a pale yellow solid, m.p. 185—7°.

Example 6

3-(5-Nitropyrazin-2-yl)amino-1,2,4-butanetriol

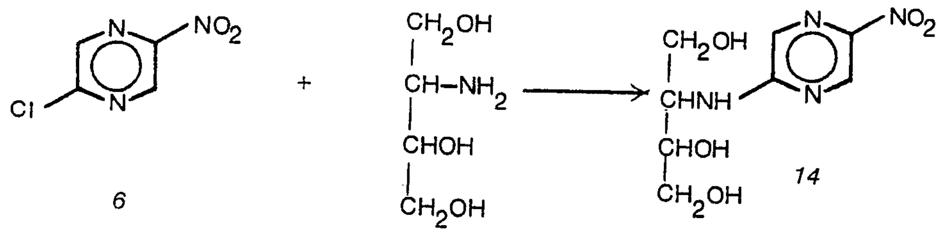

6 14

13

3-(5-Nitropyrazin-2-yl)amino-1,2,4-butanetriol (14)

To 0.5 g (0.003 mol) *6* dissolved in 15 ml acetonitrile is added a solution of 0.3 g (0.003 mol) triethylamine and 0.36 g (0.003 mol) 3-amino-1,2,4-butanetriol in 15 ml isopropanol. The reaction mixture becomes reddish in color and is stirred for 16 hours at room temperature. The solvent is removed from the rotary evaporator and the residue is purified by flash chromatography on silica gel eluting with 20% methane/chloroform. Pure *14* has Rf 0.4 and is obtained as a pale, yellow solid, m.p. 133—5°.

* Preparation 3-amino-1,2,4-butanetriol.

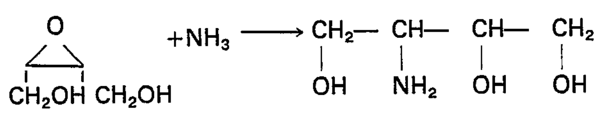

A pressure reactor is charged with 10.0 g of 1,4-butane-2,3-epoxide, 30 ml liquid ammonia and 100 ml EtOH. The reaction vessel is sealed and heated at 100° for 10 hrs.

The reaction mixture is then stripped on the rotary evaporator to leave a clear, oil which is triturated with ether to afford the desired product as a viscous oil.

Example 7

Methyl 6-(2,3-dihydroxypropyl)amino-3-nitropyrazinoate

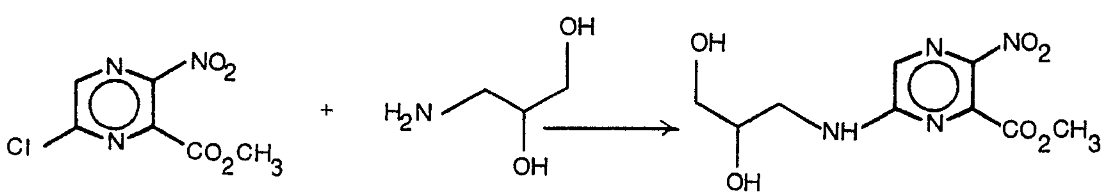

9 10 15

Methyl 6-(2,3-dihydroxypropyl)amino-3-nitropyrazinoate (15)

To 1.0 g (0.004 mol) *9* in 15 ml isopropanol is added 0.36 g (0.004 mol) *10* and 0.4 g (0.004 mol)

triethylamine at room temperature. The reaction mixture becomes reddish in color and is stirred at room temperature for 16 hours. The solvent is removed on the rotary evaporator and the residue is purified by flash chromatography on silica gel followed by elution with 10% methanol/chloroform. Pure *15* has Rf 0.4 and is obtained as a yellow solid, m.p. 107—9°.

**Claims**

1. A nitropyrazine compound of the formula I

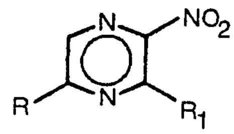

I

wherein R and $R_1$ are selected from

a) hydrogen;

b) a carboalkoxy group in which the alkoxy group contains from 1—3 carbon atoms

c) a group having the formula

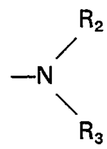

wherein

$R_2$ and $R_3$ are each selected from hydrogen and lower alkyl substituents of from 1—6 carbon atoms, said alkyl substituent having from 1—4 of the alkyl carbons substituted with a hydroxy or methoxy substituent and

d) halogen provided that at least one of the radicals R and $R_1$ is halogen or one of the specified amino groups.

2. A nitropyrazine compound according to claim 1 having the formula Ia

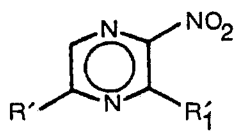

Ia

wherein R′ and $R'_1$ are selected from

a) hydrogen;

b) a carboalkoxy in which the alkoxy group contains from 1—3 carbon atoms;

c) a group having the formula

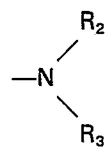

wherein

$R_2$ and $R_3$ are each selected from hydrogen and lower alkyl substituents of from 1 to 6 carbon atoms, said alkyl substituent having from 1 to 4 of the alkyl carbons substituted with a hydroxy or methoxy substituent wherein a hydroxy substituent is preferred, provided that at least one of the radicals R′ and $R'_1$ is one of the specified amino groups.

3. A compound according to claim 2 which is 2-(2,3-dihydroxypropyl)amino-3-nitropyrazine,

4. A compound according to claim 2 which is 2-(2,3-dihdyroxypropyl)amino-5-nitropyrazine,

5. A compound according to claim 2 which is 3-(5-nitropyrazin-2-yl)amino-1,2,4-butanetriol.

6. A compound according to claim 2 which is methyl 6-(2,3-dihydroxypropyl)amino-3-nitropyrazinoate.

7. A nitropyrazine compound according to claim 1 characterized in that it has the formula Ib

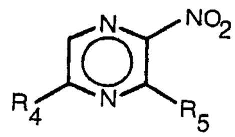

Ib

wherein

$R_4$ and $R_5$ are each selected from hydrogen, halogen and carboalkoxy in which the alkoxy group contains from 1—3 carbon atoms, provided that at least one of the radicals $R_4$ and $R_5$ is halogen.

8. A nitropyrazine compound according to claim 7 wherein in formula Ib the halogen is chloro and which nitropyrazine is preferably 3-chloro-2-nitropyrazine or 2-chloro-5-nitropyrazine or methyl 6-chloro-3-nitropyrazinoate.

9. A process for the preparation of a compound of formula I

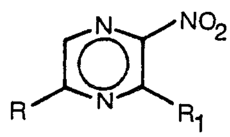

I

according to claim 1, wherein R and $R_1$ are as defined in claim 1, characterized in that a compound of formula III

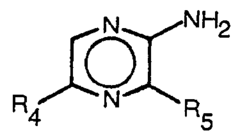

III

wherein

$R_4$ and $R_5$ are each selected from hydrogen, halogen and carboalkoxy groups, in which the alkox group contains 1—3 carbon atoms provided that at least one of the radicals $R_4$ and $R_5$ is halogen, is treate with dimethyl sulfoxide and trifluoromethane sulfonic anhydride to produce a sulfinile compound of th formula IV

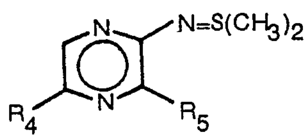

IV

which is oxidized to produce a nitropyrazine compound of the formula Ib

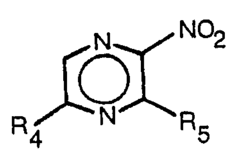

Ib

wherein $R_4$ and $R_5$ has the same meaning as in formula III and said nitropyrazine compound of formula Ib is isolated or further reacted with ammonia or a substituted amine of formula II

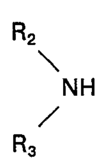

II

wherein

$R_2$ and $R_3$ are each selected from hydrogen and lower alkyl substituents of from 1—6 carbon atoms, said alkyl substituent having from 1—4 of the alkyl carbons substituted with a hydroxy or methoxy

substituent, to produce a compound of formula I in which at least one of the substituents R and $R_1$ is a group of formula

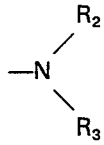

wherein

$R_2$ and $R_3$ have the same meaning as in formula II.

10. Pharmaceutical composition for enhancing the therapeutic effect of radiation characterized in that it contains as pharmaceutically active ingredient a compound of formula Ia according to patent claim 2.

11. A pharmaceutical composition according to claim 10, characterized in that it contains as further ingredient a pharmaceutically acceptable carrier.

**Patentansprüche**

1. Nitropyrazinverbindung der Formel I

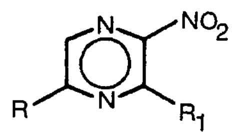

I

worin R und $R_1$ ausgewählt sind aus

a) Wasserstoff;

b) einer Carboalkoxygruppe, worin die Alkoxygruppe 1 bis 3 Kohlenstoffatome enthält;

c) einer Gruppe mit der Formel

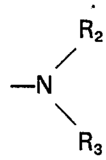

worin $R_2$ und $R_3$ jeweils ausgewählt sind aus Wasserstoff und Niederalkylsubstituenten mit 1 bis 6 Kohlenstoffatomen, wobei der Alkylsubstituent an 1 bis 4 der Alkylkohlenstoffe mit einem Hydroxy- oder Methoxysubstituenten substituiert ist, und

d) Halogen; mit der Maßgabe, daß wenigstens einer der Reste R und $R_1$ Halogen oder eine der spezifizierten Aminogruppen ist.

2. Nitropyrazinverbindung nach Anspruch 1 mit der Formel Ia

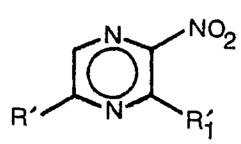

Ia

worin R' und $R'_1$ ausgewählt sind aus

a) Wasserstoff;

b) Carboalkoxy, worin die Alkoxygruppe 1 bis 3 Kohlenstoffatome enthält;

c) einer Gruppe mit der Formel

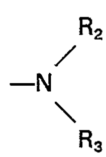

worin $R_2$ und $R_3$ jeweils ausgewählt sind aus Wasserstoff und Niederalkylsubstituenten mit 1 bis 6 Kohlenstoffatomen, wobei der Alkylsubstituent an 1 bis 4 der Alkylkohlenstoffe mit einem Hydroxy- oder Methoxysubstituenten substituiert ist, wovon ein Hydroxysubstituent bevorzugt ist, mit der Maßgabe daß

wenigstens einer der Reste R' und $R'_1$ eine der spezifierten Aminogruppen ist.

3. Verbindung nach Anspruch 2, welche 2-(2,3-Dihydroxypropyl)amino-3-nitropyrazin ist.
4. Verbindung nach Anspruch 2, welche 2-(2,3-Dihydroxypropyl)amino-5-nitropyrazin ist.
5. Verbindung nach Anspruch 2, welche 3-(5-Nitropyrazin-2-yl)amino-1,2,4-butantriol ist.
6. Verbindung nach Anspruch 2, welche Methyl-6-(2,3-dihydroxypropyl)amino-3-nitropyrazinoat ist.
7. Nitropyrazinverbindung nach Anspruch 1, dadurch gekennzeichnet, daß sie die Formel Ib aufweist

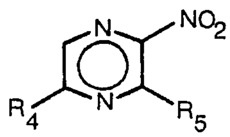

Ib

worin $R_4$ und $R_5$ jeweils ausgewählt sind aus Wasserstoff, Halogen und Carboalkoxy, worin die Alkoxygruppe 1 bis 3 Kohlenstoffatome enthält, mit der Maßgabe, daß wenigstens einer der Reste $R_4$ und $R_5$ Halolgen ist.

8. Nitropyrazinverbindung nach Anspruch 7, worin in Formel Ib das Halogen Chlor ist und welches Nitropyrazin vorzugsweise 3-Chlor-2-nitropyrazin oder 2-Chlor-5-nitropyrazin oder Methyl-6-chlor-3-nitropyrazinoat ist.

9. Verfahren zur Herstellung einer Verbindung der Formel

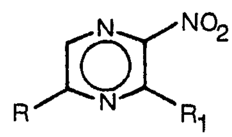

I

nach Anspruch 1, worin R und $R_1$ wie in Anspruch 1, definiert sind, dadurch gekennzeichnet, daß eine Verbindung der Formel III

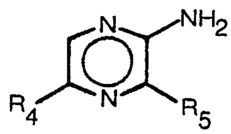

III

worin $R_4$ und $R_5$ jeweils ausgewählt sind aus Wasserstoff, Halogen und Carboalkoxygruppen, worin die Alkoxygruppe 1 bis 3 Kohlenstoffatome enthält, mit der Maßgabe, daß wenigstens einer der Reste $R_4$ und $R_5$ Halogen ist, mit Dimethylsulfoxid und Trifluormethansulfonsäureanhyrid unter Bildung einer Sulfinylverbindung der Formel IV behandelt wird

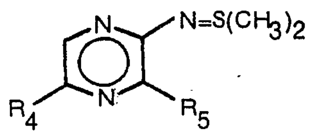

IV

welche unter Bildung einer Nitropyrazinverbindung der Formel Ib oxidiert wird

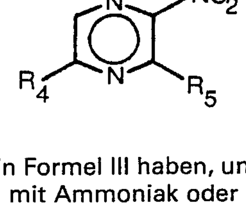

Ib

worin $R_4$ und $R_5$ gleiche Bedeutung wie in Formel III haben, und die Nitropyrazinverbindung der Formel Ib isoliert wird oder weiter umgesetzt wird mit Ammoniak oder einem substituierten Amin der Formel II

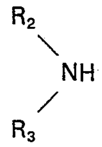

II

worin $R_2$ und $R_3$ jeweils ausgewählt sind aus Wasserstoff und Niederalkylsubstituenten mit 1 bis 6 Kohlenstoffatomen, wobei der Alkylsubstituant an 1 bis 4 der Alkylkohlenstoffe mit einem Hydroxy- oder Methoxysubstituenten substituiert ist, unter Bildung einer Verbindung der Formel I, worin wenigstens einer der Substituenten R und $R_1$ eine Gruppe der Formel ist

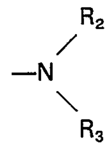

worin $R_2$ und $R_3$ die gleiche Bedeutung haben wie im Amin der Formel II.

10. Pharmazeutische Zusammensetzung zur Verstärkung des therapeutischen Effekts von Strahlung, dadurch gekennzeichnet, daß sie als pharmazeutisch aktiven Bestandteil eine Verbindung der Formel Ia gemäß Patentanspruch 2 enthält.

11. Pharmazeutische Zusammensetzung nach Anspruch 10, dadurch gekennzeichnet, daß sie als weiteren Bestandteil einen pharmazeutisch annehmbaren Träger enthält.

**Revendications**

1. Nitropyrazine de formule

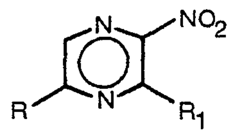

I

dans laquelle R et $R_1$ sont choisis parmi

a) l'atome d'hydrogène

b) un groupe carboalcoxy dans lequel le groupe alcoxy contient de 1 à 3 atomes de carbone,

c) un groupe ayant la formule

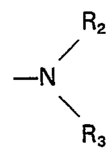

dans laquelle $R_2$ et $R_3$ sont chacun choisi parmi l'atome d'hydrogène et des substituants alkyle inférieur avec de 1 à 6 atomes de carbone, lesdits substituants alkyle ayant de 1 à 4 des carbones alkyle substitués par un substituant hydroxyle ou méthoxy, et

d) une atome d'halogène à condition qu'au moins un des radicaux R et $R_1$ soit un atome d'halogène ou un des groupes amino désignés.

2. Nitropyrazine selon la revendication 1, répondant à la formule Ia

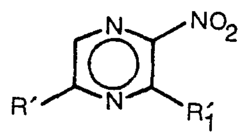

Ia

dans laquelle R' et $R_1'$ sont choisis parmi

a) l'atome d'hydrogène;

b) un groupe carboalcoxy dans lequel le groupe alcoxy contient de 1 à 3 atomes de carbone;

c) un groupe de formule

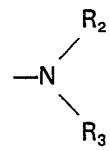

dans laquelle $R_2$ et $R_3$ sont chacun choisi parmi l'atome d'hydrogène et des substituants alkyle inférieur avec de 1 à 6 atomes de carbone, lesdits substituants alkyle ayant de 1 à 4 des carbones alkyle substitués par un substituant hydroxyle ou méthoxy, un substituant hydroxyle étant preféré, à condition qu'au moins un des radicaux R' et $R'_1$ soit un des groupes amino désignés.

3. Composé selon la revendication 2, qui est la 2-(2,3-dihydroxypropyl)amino-3-nitropyrazine.

4. Composé selon la revendication 2, qui est la 2-(2,3-dihydroxypropyl)amino-5-nitropyrazine.

5. Composé selon la revendication 2, qui est le 3-(5-nitropyrazine-2-yl)amino-1,2,4,-butanetriol.

6. Composé selon la revendication 2, qui est le 6-(2,3-dihydroxypropyl)amino-3-nitropyrazinoate de méthyle.

7. Nitropyrazine selon la revendication 1, caractérisée en ce qu'elle a la formule Ib

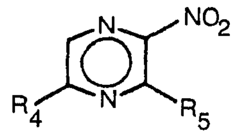

Ib

dans laquelle $R_4$ et $R_5$ sont chacun choisis parmi l'atome d'hydrogène, un atome d'halogène et un groupe carboalcoxy dans lequel le groupe alcoxy contient de 1 à 3 atomes de carbone, à condition qu'au moins un des radicaux $R_4$ et $R_5$ soit un atome d'halogène.

8. Nitropyrazine selon la revendication 7, pour laquelle dans la formule Ib l'atome d'halogène est l'atome de chlore, et cette nitropyrazine étant de préférence la 3-chloro-2-nitropyrazine ou la 2-chloro-5-nitropyrazine ou le 6-chloro-3-nitropyrazinoate de méthyle.

9. Procédé pour la préparation d'un composé de formule I

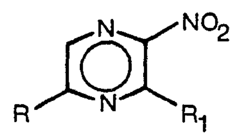

I

selon la revendication I, dans laquelle R et $R_1$ sont tels que définis dans la revendication 1 caractérisé en ce qu'on traite un composé de formule III

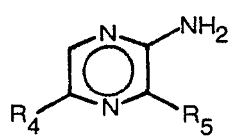

III

dans laquelle $R_4$ et $R_5$ sont chacun choisis parmi l'atome d'hydrogène, un atome d'halogène et un groupe carboalcoxy dans lequel le groupe alcoxy contient de 1 à 3 atomes de carbone, à condition qu'au moins un des radicaux $R_4$ et $R_5$ soit un atome d'halogène, avec le diméthylsulfoxyde et l'anhydride trifluorométhane-sulfonique pour produire un composé sulfilimine de formule IV

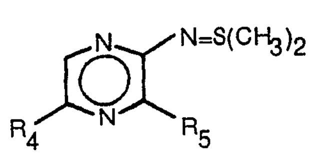

IV

que l'on oxyde pour produire un composé de nitropyrazine de formule Ib

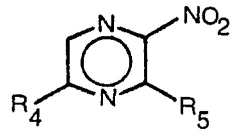

Ib

dans laquelle $R_4$ et $R_5$ ont la même signification que dans la formule III et que l'on isole ledit composé de nitropyrazine de formule Ib ou qu'on le fait ensuite réagir avec l'ammoniac ou une amine substituée de formule II

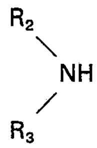

II

dans laquelle $R_2$ et $R_3$ sont chacun choisi parmi l'atome d'hydrogène et des substituants alkyle inférieur avec de 1 à 6 atomes de carbone, lesdits substituants alkyle ayant de 1 à 4 des carbones alkyle substitués par un substituant hydroxyle ou méthoxy, pour produire un composé de formule I dans laquelle au moins un des substituants R et $R_1$ est un groupe de formule

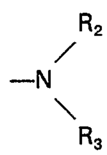

dans laquelle $R_2$ et $R_3$ ont la même signification que dans l'amine de formule II.

10. Composition pharmaceutique pour accentuer l'effet thérapeutique de l'irradiation, caractérisée en ce qu'elle contient, à titre d'ingrédient pharmaceutiquement actif, un composé de formule Ia selon la revendication 2.

11. Composition pharmaceutique selon la revendication 10, caractérisée en ce qu'elle contient à titre d'ingrédient supplémentaire, un véhicule pharmaceutiquement acceptable.